# EUROPEAN PATENT SPECIFICATION

(11) **EP 0 673 921 B1**
(45) Date of publication and mention of the grant of the patent: **22.07.1998**
(21) Application number: 95102985.9
(22) Date of filing: 02.03.1995
(51) Int. Cl.: C07C 227/40, C07C 229/36, C07K 5/06

(54) **Process for recovering L-phenylalanine**
Verfahren zur Rückgewinnung von L-Phenylalanin
Procédé pour la récupération de L'phénylalanine

(30) Priority: 24.03.1994 JP 53742/94
(43) Date of publication of application: 27.09.1995
(73) Proprietor: Ajinomoto Co., Inc., Tokyo 104 (JP)
(72) Inventor: Hijiya, Toyoto, c/o Central Research Lab., Kawasaki-shi, Kanagawa-ken (JP); Mochizuki, Chiaki, c/o Central Research Lab., Kawasaki-shi, Kanagawa-ken (JP); Takemoto, Tadashi, c/o Central Research Lab., Kawasaki-shi, Kanagawa-ken (JP); Nagashima, Kazutaka, c/o Tokai Plant, Yokkaichi-shi, Mie-ken (JP)
(74) Representative: Strehl Schübel-Hopf & Partner

(56) References cited:
- EP-A- 0 055 612
- EP-A- 0 526 854
- FR-A- 2 040 473
- CHEMICAL ABSTRACTS, vol. 109, no. 23, 5 December 1988, Columbus, Ohio, US; abstract no. 211485u, R. MITA ET. AL. 'Recovery of L-phenylalanine and L-aspartic acid from a process for aspartame manufacture.' page 710 ;column 1 ; & JP-A-63 145 298 (MITSUI TOATSU CHEMICALS INC.)

## Description

### Field of the Invention

This invention relates to a process for producing α-L-aspartyl-L-phenylalanine methyl ester (hereinafter abbreviated to α-APM), which is about 200 times sweeter than sucrose and is extensively required for a sweetener due to its good sweet taste and low calorie content. More particularly, this invention relates to a process for recovering L-phenylalanine (hereinafter abbreviated as L-Phe) from the side flow generated from a process for producing α-APM.

### Description of Prior Art

Various processes for producing α-APM are known, for example a process which comprises a reaction between N-protected L-aspartic anhydride and L-phenylalanine methyl ester (US Patent 3786039), a process which comprises a reaction between N-protected L-aspartic anhydride and L-Phe (US Patent 4173562), a process which comprises the enzymatic condensation of N-benzyloxycarbonyl-L-aspartic acid and L-phenylalanine methyl ester (Japanese Patent Laying-Open No. 92729/1978).

However, to make them low cost industrial processes, it is required to recover and reuse L-Phe and L-aspartic acid (hereinafter abbreviated as L-Asp) from a side flow obtained in the production of α-APM, for example, the mother liquor of the crystallization of α-APM or the mother liquor of the crystallization of α-APM·HCl, since these side flows contain a significant amount of L-phenylalanine derivatives, L-aspartic acid derivatives, β-L-aspartyl-L-phenylalanine methyl ester and α-APM. It is particularly very important to efficiently recover L-Phe, which is an expensive amino acid.

As a process for recovering L-Phe from a side flow generated from a process for production of α-APM, a process disclosed in Japanese Patent Laying-Open No. 97812/1973 is known. The process comprises hydrolyzing a solution containing β-L-aspartyl-L-phenylalanine methyl ester in the presence of a mineral acid, adjusting the pH to around the isoelectric point of L-Phe, i.e., about 6, by adding aqueous alkali to the reaction solution to crystallize and separate L-Phe. To improve yield of L-Phe during this crystallization step, it is required to increase the concentration of L-Phe in the hydrolyzate. However, by this neutralization process, L-Asp is also separated out during the step to increase the pH by adding aqueous alkali. Particularly, in the range around pH 1 at which L-Asp begins to crystallize, the stirring ability of the crystallization slurry becomes extremely worse. The fact means that an increased concentration of L-Phe results in an increased concentration of L-Asp which exists in an almost equimolar amount to L-Phe. Accordingly, the concentration of the slurry becomes too high in the pH range around 1. In some cases, the whole or a part of the crystallization slurry may be solidified and cannot be stirred. Accordingly, to improve the recovery of L-Phe, it is required to improve the stirring ability of the slurry during adjustment of pH, which is a bottle neck.

Moreover, in this neutralization method, the deterioration of the stirring ability results in smaller L-Phe crystals. Further, the L-Phe crystals obtained after separation have a high water content. Using L-Phe with high water content as a raw material for production of α-APM disadvantageously requires an extremely high load for drying when L-Phe is recycled in the process described in US Patent No. 4173562. When L-phenylalanine is converted into the methyl ester thereof for recycle, for example, in processes disclosed in US Patent No. 3786039 and Japanese Patent Laying-Open No. 92729/1978, the yield of ester production may be lowered because esterification is an equilibrium reaction with hydrolysis.

EP-A-0 526 854 relates to a method for the recovery of α-L-aspartyl-L-phenylalanine methyl ester, L-phenylalanine and L-aspartic acid from the mother liquor obtained by the solid-liquid separation of a suspension of α-L-aspartyl-L-phenylalanine methyl ester crystals. The recovery of L-phenylalanine by addition of an acidic phenylalanine solution to a slurry of phenylalanine maintained in the pH range 3-8 by addition of alkali is neither mentioned nor suggested.

### Problem to be solved by the Invention

The problem to be solved by the invention is to establish an industrial process to recover L-Phe with a lower water content at high yield when L-Phe is crystallized and recovered by neutralization from the hydrolyzed reaction solution of a side flow, generated from α-APM, in the presence of a mineral acid.

### Means to solve the Problem

The present inventors have studied intensively to solve the above problems. Unexpectedly, we have found that a process, wherein a hydrolyzate and aqueous alkali are simultaneously added to L-Phe slurry while the pH of the slurry is maintained at 5 - 6, provides a slurry with remarkably improved fluidity compared with that obtained by the conventional process wherein the pH is adjusted by adding aqueous alkali to a solution, obtained by hydrolysis of a side flow of α-APM with a mineral acid, in the presence of a mineral acid. The present inventors have further studied intensively. As the result, they have found when a hydrolyzate having a concentration of L-Phe of not less than 8 g/dl and aqueous alkali are simultaneously added to the L-Phe slurry while the pH of the slurry is maintained at about 3 - 8, L-Phe with less water content can be recovered at high yield, and have attained the present invention. That is, according to this process, a hydrolyzate of strong acidity is rapidly brought to pH 3 to 8, at which stirring ability becomes better, without experiencing a pH about 1, at which stirring ability of the crystallization slurry becomes worse. By such addition, good slurry properties can be maintained even using a hydrolyzate with such high L-Phe concentration that the fluidity of the slurry may become extremely worse and the slurry will solidify according to the conventional method. Moreover, the crystallization yield of L-Phe from the resulting crystallization slurry can be improved. In addition, L-Phe crystals obtained by this method have a remarkably reduced water content compared with those obtained by the conventional method.

Typical examples of the side flow obtained from the process for the production of α-APM, which is an object of the present invention, include, for example,
(i) a mother liquor obtained by crystallizing and separating α-APM from water, which is obtained by condensing N-benzyloxycarbonyl-L-aspartic anhydride and L-phenylalanine methyl ester, followed by deprotection upon reduction (US Patent 3786039);
(ii) a mother liquor obtained by separating α-APM·HCl precipitated upon treatment with methanol and hydrochloric acid at high concentration of a condensation product of N-formyl-L-aspartic anhydride and L-phenylalanine methyl ester (US Patent 4684745);
(iii) a mother liquor crystallized and separated as α-APM·HCl after esterification of α-L-aspartyl-L-phenylalanine with water, methanol and hydrochloric acid (US Patent 4173562).

The mineral acids used in the present invention include hydrochloric, sulfuric, phosphoric acid and the like. Considering the easiness of removal of their salts generated by the adjustment of pH after hydrolysis, hydrochloric acid is desirable. During hydrolysis, the concentration of these acids is preferably 1 - 12 N, particularly 2 - 6 N. The amount used is desirably more than 1 mole, particularly 1.5 to 10 mole based on 1 mole of amino acid and amino acid residue contained in said side flow.

The concentration of L-Phe in the hydrolyzate is not particularly limited as long as said hydrolysate is obtained by hydrolysis at the above acid concentration. The present invention is effective for a hydrolyzate of the concentration above 8 g/dl At such concentration, the reaction solution is readily solidified according to the conventional neutralization.

The temperature for hydrolysis is 60 - 120°C. Especially, 90 - 110°C is desirable. The reaction time may not be limited because it varies depending on various factors. However, too long reaction time promotes racemization. Accordingly, a reaction time within 20 hours is sufficient.

The thus obtained hydrolyzate is added to the crystallization slurry of L-Phe. When a hydrolyzate of high L-Phe concentration is prepared, the hydrolyzate is desirably heated during the addition to prevent crystallization.

During the addition of the hydrolyzate, the L-Phe slurry is maintained within the pH range of 3 - 8 by adding aqueous alkali. To improve the crystallization yield of L-Phe, it is desirable to adjust the pH of the obtained L-Phe slurry finally to 5 - 6 before solid-liquid separation, from the viewpoint of isoelectric point of L-Phe.

Alkali used for pH adjustment includes sodium hydroxide, potassium hydroxide, sodium carbonate, ammonia and the like. Sodium hydroxide is most preferable from the viewpoint of cost. The alkali may be directly added. However, it is desirable to added in the form of an aqueous solution because of easiness of pH adjustment.

The temperature of the crystallization slurry for pH adjustment is not particularly limited. The adjustment may be carried out at 0 - 100°C, desirably at 0 - 80°C. When it is carried out at a temperature above 10°C, it is desirable to cool the crystallization slurry finally below 10°C after the addition of the hydrolyzate is completed, because the yield is improved by lowering the solubility of L-Phe.

The rate of addition of this hydrolyzate has no particular effect on the stirring ability of the slurry. However, in order to control the increase of temperature due to the heat generated by neutralization of the strongly acidic hydrolyzate with aqueous alkali, the whole amount of the hydrolyzate is desirably added over a period of one hour or more.

In the slurry containing L-Phe to which the hydrolyzate and aqueous alkali are added, the concentration of L-Phe is not particularly limited. For example, water or an aqueous solution containing L-Phe may be used instead of L-Phe slurry. In such cases, as hydrolyzate and aqueous alkali are added, the concentration of L-Phe in the solution is increased, and L-Phe is separated out as crystals beyond its solubility. Once L-Phe is separated out, such addition is the same as that to L-Phe slurry.

Accordingly, the volume of L-Phe slurry to which the hydrolyzate is added is not particularly limited. In industrial scale, any liquid volume may be employed, in which stirring using, for example, stirring blade attached to a L-Phe slurry tank can be performed, and a pH meter functions effectively.

The present invention will be explained in detail in the following examples.

### Example 1

A mother liquor of crystallization of α-APM hydrochloride was heated at 105°C for 7 hours to obtain a hydrolyzate solution. According to the analysis using an amino acid analyzer, the concentrations of L-Phe and L-Asp were 16.41 g/dl and 14.91 g/dl, respectively. The HCl concentration was 3.5 N. This hydrolyzate (15 ml) was adjusted to pH 5.6 with 48 % NaOH to prepare an L-Phe slurry. This L-Phe slurry was stirred at 40°C, to which was added the same hydrolyzate (685 ml) over 10 hours. During this step, the pH of the crystallization slurry was maintained between 5 and 7 by addition of 48 % NaOH. The pH after completion of the addition of the hydrolyzate was adjusted to 5.6. The stirring ability during crystallization was not accompanied with any particular problems, providing a good slurry. During this step, the temperature was maintained at 38 - 45°C. Then, the obtained L-Phe slurry was stirred at 6°C for 24 hours, then separated by suction filtration. The crystals were washed with water (90 ml).

According to the analysis using an amino acid analyzer, 138.7 g of the resulting wet crystals contained 109.0 g of L-Phe. Yield, 94.9 %. This wet crystals were dried at 105°C for 4 hours. The loss on drying was 20.5 %.

### Comparative Example 1

The hydrolyzate obtained in Example 1 (300 ml) was stirred and heated in a 500 ml flask at 70°C, to which was added 48 % NaOH. Around pH 0.7, crystals started to separate out, resulting in extremely bad stirring ability.

In addition, the peripheral portion was solidified and could not be stirred. 48 % NaOH was further added to bring the pH to 5.6. The peripheral portions remained solidified. The peripheral solid was broken using a spatula and heated to 90°C to become a slurry. This slurry was cooled to 5°C, stirred overnight, separated by suction filtration, and the resulting crystals were washed with water (40 ml). The resulting wet crystals (75.6 g) contained 46.1 g of L-Phe. Yield, 93.6 %. Loss on drying, 37.2 %.

### Comparative Example 2

In the same manner as Example 1, a hydrolyzate solution was obtained from a mother liquor of α-APM·HCl crystallization. According to the analysis using an amino acid analyzer, the concentration of L-Phe is 17.0 g/dl. This hydrolyzate solution was diluted by addition of water to prepare 150 ml hydrolyzate solutions with L-Phe concentrations of 6, 8, 10, 13 and 15 g/dl, respectively. Each solution was charged in a 300 ml flask and stirred at 40°C. 48 % NaOH was added to the solution and stirring ability of the slurry was examined until the pH reached 5.6. A good slurry state was maintained only when a hydrolyzate having an L-Phe concentration of 6 g/dl was used. When a hydrolyzate of an L-Phe concentration above 8 g/dl was used, the peripheral portion was solidified around pH 1.

### Example 2

35 % HCl (140 g) was added to a mother liquor of the α-APM crystallization (25 l) and concentrated to 0.8 l under reduced pressure. 35 % HCl (HCl concentration, 3.5 N) was added to this concentrate and heated at 105°C for 7 hours to obtain a hydrolyzate. According to the analysis using an amino acid analyzer, the concentrations of L-Phe and L-Asp were 18.35 g/dl and 15.29 g/dl, respectively. The separated mother liquor obtained in Example 1 (20 ml, containing 0.1 g of L-Phe) was stirred at 70°C, to which was added the hydrolyzate (600 ml) over 3 hours. During this addition, the pH of the crystallization slurry was maintained at 5 - 7 by addition of 48 % NaOH. After the addition of the hydrolyzate was completed, the pH was adjusted to 5.6. The stirring ability during the crystallization was accompanied with no particular problem, providing a good slurry. The temperature during this addition was maintained at 68 - 73°C . Thereafter, the resulting L-Phe slurry was stirred at 6°C for 24 hours, separated by suction filtration, and the crystals were washed with water (90 ml).

The resulting wet crystals (128.5 g) contained 104.9 g of L-Phe. Yield, 95.3 %. Loss on drying, 16.1 %.

### Comparative Example 3

The hydrolyzate obtained in Example 2 (250 ml) was charged in a 500 ml flask, heated and stirred at 70°C, to which was added 48 % NaOH. Around pH 0.4, crystals suddenly began to separate out, resulting in an extremely bad stirring ability and a solidified peripheral portion. Hot water (150 ml) was added to the flask. After a while, the solid became a slurry and could be stirred. Then, 48 % NaOH was added to adjust the pH to 5.6. Subsequently, the reaction was cooled to 5°C, maintained at the temperature for 3 hours, then separated by suction filtration. The crystals were washed with water (45 ml).

The resulting wet crystals (65.1 g) contained 41.1 g of L-Phe. Yield, 89.7 %. Loss on drying, 34.8 %.

### Example 3

In the same manner as in Example 1, a hydrolyzate was obtained from the mother liquor of the α-APM·HCl crystallization. According to the analysis using an amino acid analyzer, the concentrations of L-Phe and L-Asp were 14.61 g/dl and 12.20 g/dl, respectively. The concentration of HCl was 4.0 N. The hydrolyzate (20 ml) was adjusted to pH 3.5 with 48 % NaOH to prepare an L-Phe slurry. This slurry was stirred at 40°C, to which was added the same hydrolyzate (480 ml) over 3 hours. During this addition, the pH of the crystallization slurry was maintained within 3.4 - 4.0 by addition of 48 % NaOH. After the addition of hydrolyzate was completed, stirring was continued at pH 3.5 for an hour. Then, the pH was adjusted to 5.6 with 48 % NaOH. The stirring ability during the crystallization was accompanied with no particular problems, providing a good slurry. The temperature during this step was maintained at 38 - 45°C. Thereafter, the resulting L-Phe slurry was stirred at 6°C for 5 hours, separated by suction filtration, and the crystals were washed with water (50 ml).

The resulting wet crystals (79.0 g) contained 68.1 g of L-Phe. Yield, 93.2 %. Loss on drying, 13.2 %.

### Example 4

In the same manner as in Example 1, a hydrolyzate was obtained from the mother liquor of the α-APM·HCl crystallization. According to the analysis using an amino acid analyzer, the concentration of L-Phe was 16.5 g/dl. A 1 l cylindrical vessel having an overflow hole in the portion corresponding to 400 ml was equipped with a stirring blade, a thermometer, a pH meter and a dropping funnel containing 48 % aqueous NaOH for neutralization. The hydrolyzate was continuously added into the cylindrical vessel at a rate of 100 ml/hr. During this step, the temperature of the solution was maintained at 37 - 43°C, and the pH was controlled at 5.3 - 5.6 by addition of 48 % NaOH. Immediately after the neutralization, the hydrolyzate became a slurry. On 3 1/4 hours after beginning of the addition, the slurry began to overflow from the overflow hole. This slurry was collected in an another vessel. This operation was continued for 7 hours. The slurry in the cylindrical vessel was cooled in a refrigerator overnight, then separated by suction filtration. The loss on drying of the resulting crystals was 11.6 %.

### Example 5

The procedure of Example 4 was continued for 6 hours, except that a hydrolyzate of L-Phe concentration of 14.9 g/dl was used and the pH was controlled to 4.2 - 4.5. Further, the resulting slurry was adjusted to pH 5.6, then crystallized in a refrigerator overnight and separated by suction filtration. The loss on drying of the resulting crystals was 12.3 %.

### Example 6

The procedure of Example 5 was continued for 6 hours, except that the hydrolyzate was added at a rate of 200 ml/hr and the pH was controlled to 3.3 - 3.5. Further, the resulting slurry was adjusted to pH 5.6, then crystallized in a refrigerator overnight and separated by suction filtration. The loss on drying of the resulting crystals was 18.7 %.

### Advantages of the Invention

L-Phenylalanine with lower water content can be crystallized at a high yield by recovering L-phenylalanine by crystallization from a reaction mixture obtained by hydrolysis of at least one side flow obtained from the production of α-L-aspartyl-L-phenylalanine methyl ester in the presence of a mineral acid, without difficulties being caused in the stirring of the crystallization slurry.

## Claims

1. A process for recovering L-phenylalanine by crystallization from the reaction mixture of the hydrolysis of at least one of the side flows from α-L-aspartyl-L-phenylalanine methyl ester production in the presence of a mineral acid, characterised in that the concentration of L-phenylalanine in the hydrolyzed reaction mixture is not less than 8 g/dl and that the hydrolyzed reaction mixture is added to a crystallization slurry containing L-phenylalanine while the pH of the slurry is maintained at 3 to 8 by adding aqueous alkali to the slurry.

2. A process according to claim 1, wherein the mineral acid is hydrochloric acid.

## Patentansprüche

1. Verfahren zur Gewinnung von L-Phenylalanin durch Kristallisation aus dem Reaktionsgemisch der Hydrolyse mindestens eines der Nebenströme aus der Herstellung von α-L-Aspartyl-L-phenylalanin-methylester in Gegenwart einer Mineralsäure, dadurch gekennzeichnet, daß die Konzentration an L-Phenylalanin in dem hydrolysierten Reaktionsgemisch nicht weniger als 8 g/dl betragt und daß das hydrolysierte Reationsgemisch zu einer Kristallisationsaufschlämmung gegeben wird, die L-Phenylalanin enthält, während der pH-Wert der Aufschlämmung bei 3 bis 8 durch Zugabe von wäßrigem Alkali zu der Aufschlämmung gehalten wird.

2. Verfahren nach Anspruch 1, wobei es sich bei der Mineralsäure um Chlorwasserstoffsäure handelt.

## Revendications

1. Procédé pour la récupération de L-phénylalanine par cristallisation à partir du mélange réactionnel de l'hydrolyse d'au moins un des écoulements secondaires de la production d'ester méthylique de α-L-aspartyl-L-phénylalanine en présence d'un acide minéral, caractérisé en ce que la concentration en L-phénylalanine dans le mélange réactionnel hydrolysé n'est pas inférieure à 8 g/dl et en ce que le mélange réactionnel hydrolysé est ajouté à une suspension de cristallisation contenant de la L-phénylalanine alors que le pH de la suspension est maintenu à de 3 à 8 par addition d'un alcali aqueux à la suspension.

2. Procédé selon la revendication 1, dans lequel l'acide minéral est l'acide chlorhydrique.
